# FASCICULE DE BREVET EUROPEEN

(11) **EP 3 164 223 B1**
(45) Date de publication et mention de la délivrance du brevet: **12.09.2018**
(21) Numéro de dépôt: 15753094.0
(22) Date de dépôt: 02.07.2015
(51) Int. Cl.: B05B 11/02, A61M 11/00, A61M 15/00, A61M 15/08, B05B 11/00

(54) **DISPOSITIF DE DISTRIBUTION DE PRODUIT FLUIDE**
VORRICHTUNG ZUR AUSGABE EINES FLUIDPRODUKTS
DEVICE FOR DISPENSING A FLUID PRODUCT

(30) Priorité: 04.07.2014 FR 1456492
(43) Date de publication de la demande: 10.05.2017
(73) Titulaire: APTAR France SAS, 27110 Le Neubourg (FR)
(72) Inventeur: HELDT, Frédéric, 27400 Louviers (FR); LEBEL, Baptiste, 27100 Val de Reuil (FR); PETIT, Ludovic, 27110 Vitot (FR)
(74) Mandataire: CAPRI
(86) Numéro de dépôt international: PCT/FR2015/051839
(87) Numéro de publication internationale: WO 2016/001599

(56) Documents cités:
- EP-A1- 0 546 607
- EP-A2- 1 084 763
- EP-A2- 1 447 140
- WO-A1-92/00812
- US-B1- 6 626 379

## Description

La présente invention concerne un dispositif de distribution de produit fluide, et plus particulièrement un dispositif du type unidose ou bidose, adapté à distribuer une ou deux doses de produit fluide en un seul actionnement.

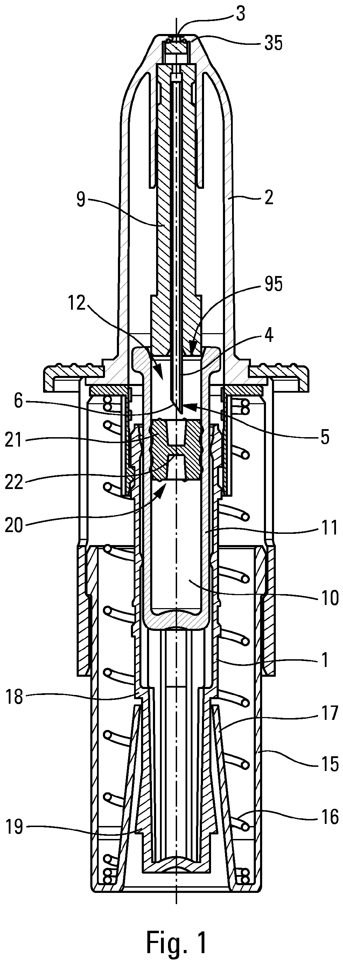

Les dispositifs du type unidoses ou bidoses sont bien connus. Comme visible sur la figure 1, ils comportent généralement un corps 1 recevant un réservoir 10 contenant une ou deux doses de produit fluide, et une tête de distribution 2 pourvue d'un orifice de distribution 3, et qui est déplaçable axialement par rapport audit corps 1 lors de l'actionnement. Le réservoir 10 est généralement formé par un tube creux borgne 11 dont l'ouverture axiale proximale 12 (par rapport à l'orifice de distribution 3) est fermée de manière étanche par un bouchon 20. La tête de distribution 2 comporte généralement une canule ou aiguille 4, de forme généralement cylindrique, reliée d'un coté audit orifice de distribution 3, et pourvue de l'autre coté d'une pointe de perçage 5 adaptée à percer ledit bouchon 20 lors de l'actionnement, le bouchon 20 se déplaçant alors dans ledit réservoir 10 pour expulser la ou les doses de produit fluide à travers ladite canule 4 vers ledit orifice de distribution 3. La canule 4 est généralement reçue fixement, notamment insérée à force dans un support de canule 9 solidaire de la tête de distribution 2. Le document EP0546607 décrit un dispositif de ce type.

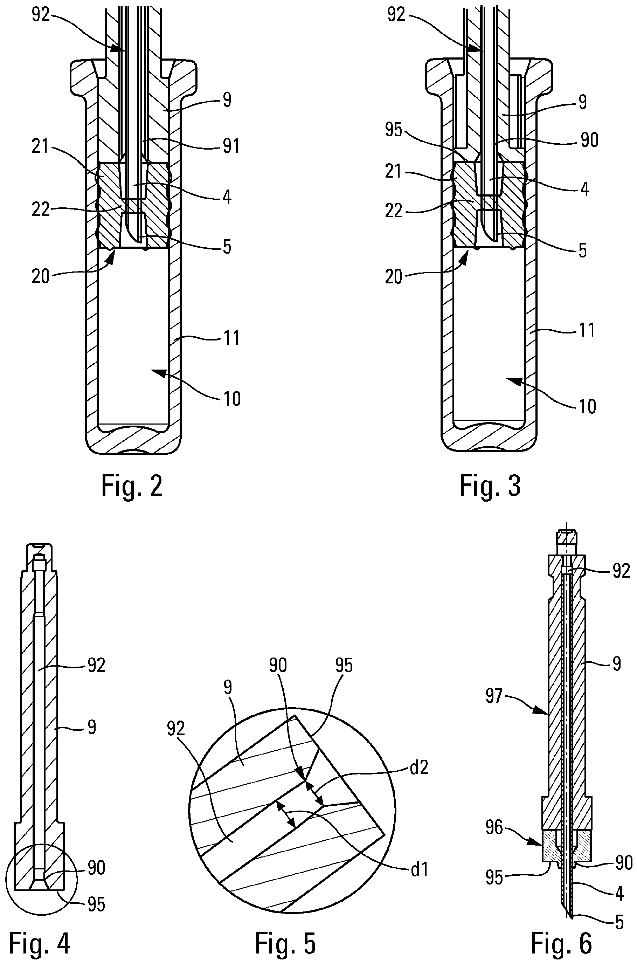

Un inconvénient avec ces dispositifs de l'art antérieur concerne les risques de fuites lors de l'actionnement, ce qui altère la dose distribuée et la reproductibilité du dosage entre différents dispositifs du même type. En particulier, comme visible sur la figure 2, le support de canule 9 comporte un alésage central 92 pourvu de nervures longitudinales 91 permettant l'insertion de la canule 4 sans trop de frottements, tout en assurant une bonne fixation de ladite canule dans le support de canule. Du liquide ayant fui hors du réservoir, par exemple lors de l'actionnement, peut alors s'écouler dans ledit support de canule 9, en particulier dans la totalité dudit alésage central 92, ce qui peut représenter un volume important.

Le document EP1447140 montre un dispositif de distribution de produit fluide selon le préambule de la revendication 1.

La présente invention a pour but de fournir un dispositif de distribution de produit fluide, notamment du type unidose ou bidose, qui ne reproduit pas les inconvénients susmentionnés.

La présente invention a aussi pour but de fournir un dispositif de distribution de produit fluide, notamment du type unidose ou bidose, qui améliore la précision du dosage et sa reproductibilité pour une pluralité de dispositifs du même type, en limitant le volume susceptibles de recevoir des fuites lors de l'actionnement.

La présente invention a également pour but de fournir un dispositif de distribution de produit fluide, notamment du type unidose ou bidose, qui est simple et peu coûteux à fabriquer et à assembler.

La présente a donc pour objet un dispositif de distribution de produit fluide comportant un corps et une tête de distribution pourvue d'un orifice de distribution et déplaçable axialement par rapport audit corps lors de l'actionnement, ledit corps recevant un réservoir contenant du produit fluide, ledit réservoir comportant un tube creux ayant une ouverture axiale proximale fermée par un bouchon adapté à coulisser de manière étanche dans ledit tube lors de l'actionnement, ladite tête de distribution comportant une canule creuse reliée d'un côté audit orifice de distribution et pourvue de l'autre côté d'une pointe de perçage pourvue d'une ouverture pour percer ledit bouchon, ladite canule étant insérée dans un support de canule solidaire de ladite tête de distribution, ledit support de canule comportant un alésage axial central dont le diamètre interne d1 est égal ou légèrement supérieur au diamètre externe de ladite canule, l'extrémité axiale distale dudit support de canule comportant une projection radiale s'étendant radialement vers l'intérieur, dont le diamètre interne d2 est très légèrement inférieur au diamètre externe de ladite canule, le diamètre interne d2 de ladite projection radiale étant inférieur au diamètre interne d1 dudit alésage axial central.

Avantageusement, après insertion de la canule dans le support de canule, ladite projection radiale coopère de manière étanche avec ladite canule.

Avantageusement, la surface radiale d'extrémité frontale de l'extrémité axiale distale du support de canule est plane et coopère, lors de l'actionnement, de manière étanche avec le bouchon.

Avantageusement, le support de canule est réalisé en deux parties, une partie supérieure avec un alésage axial central de diamètre interne constant, et une partie inférieure comportant ladite projection radiale de diamètre interne réduit.

Avantageusement, ledit réservoir est formé par un tube creux borgne.

Avantageusement, ledit réservoir contient une dose unique de produit fluide distribuée lors d'un seul actionnement du dispositif.

En variante, ledit réservoir contient deux doses de produit fluide distribuées lors de deux actionnements successifs du dispositif.

Ces caractéristiques et avantages et d'autres apparaîtront plus clairement au cours de la description détaillée suivante, faite en référence aux dessins joints, donnés à titre d'exemples non limitatifs, et sur lesquels :
- la figure 1 est une vue schématique d'un dispositif de distribution de type bidose auquel peut s'appliquer la présente invention ;
- la figure 2 est une vue de détail en section transversale d'une canule standard de l'art antérieur;
- la figure 3 est une vue similaire à celle de la figure 2, illustrant un mode de réalisation avantageux de la présente invention;
- la figure 4 est une de détail en section transversale du support de canule de la figure 3;
- la figure 5 est une vue de détail du support de canule de la figure 4; et
- la figure 6 est une vue de détail d'un support de canule selon une variante de réalisation.

La présente invention concerne plus particulièrement un dispositif du type de celui divulgué dans les documents EP1447140 et EP0546607.

Il est toutefois entendu que la présente invention ne se limite pas à ce type de dispositif, mais est au contraire applicable à tous les types de dispositifs de distribution de produit fluide, du type unidose ou bidose ou du type multidoses, comportant un réservoir obturé par un bouchon, ledit bouchon devant être percé avant ou au début de l'actionnement.

Dans la description, les termes "axial" et "radial" se réfèrent en particulier à la direction longitudinale de la canule cylindrique 4. Les termes "proximal" et "distal" se réfèrent à l'orifice de distribution 3 formé dans la tête de distribution 2.

L'invention s'applique plus particulièrement à des dispositifs du type unidose ou bidose tel que celui représenté sur la figure 1. Comme décrit précédemment, un tel dispositif comporte un corps 1 qui reçoit un réservoir 10 contenant une ou deux doses de produit fluide, et une tête de distribution 2, pourvue d'un orifice de distribution 3, est déplaçable axialement par rapport audit corps 1 lors de l'actionnement.

Le réservoir 10 contient une ou deux dose(s) de produit fluide. Avantageusement, ledit réservoir 10 est formé par un tube creux borgne 11, par exemple en verre, ayant une ouverture axiale 12 fermée par un bouchon 20, par exemple en élastomère, adapté à coulisser de manière étanche dans ledit tube 11 lors de l'actionnement. Ledit bouchon 20 comporte une partie axiale tubulaire 21 coulissant de manière étanche dans le tube 11, et une paroi transversale 22 destinée à être percée par une canule 4 lors de l'actionnement.

La tête de distribution 2 comporte généralement une canule ou aiguille 4, de forme généralement cylindrique, reliée d'un côté audit orifice de distribution 3, et pourvue de l'autre côté d'une pointe de perçage 5 adaptée à percer ledit bouchon 20 lors de l'actionnement, le bouchon 20 se déplaçant alors dans ledit réservoir 10 pour expulser la ou les doses de produit fluide à travers ladite canule 4 vers ledit orifice de distribution 3. La canule 4 est insérée dans un support de canule 9, qui lui-même est fixé dans ladite tête de distribution 2. Avantageusement, un profil de pulvérisation 35 peut être formé directement en amont de l'orifice de distribution 3, par exemple entre le fond de ladite tête de distribution 2 et l'extrémité axiale proximale dudit support de canule 9.

Un organe d'actionnement 15, connecté audit corps 1, peut être prévu pour réaliser l'actionnement. Dans l'exemple représenté sur la figure 1, l'organe d'actionnement 15 est sollicité vers sa position de repos par un ressort 16, et est déplaçable manuellement, contre la force du ressort 16, pour déplacer le corps 1 avec le réservoir 10 par rapport à la tête de distribution 2. L'exemple de la figure 1 étant un bidose, l'organe d'actionnement comporte des moyens de fractionnement de dose, formés dans cet exemple sous la forme d'une ou plusieurs pattes flexibles 17 coopérant avec des épaulements radiaux respectifs 18, 19 formés sur ledit corps 1. Ainsi, pour distribuer la première dose, la patte flexible 17 va coopérer avec l'épaulement 18, puis le ressort 16 va ramener l'organe d'actionnement 15 en position de repos, dans laquelle ladite patte flexible coopérera avec le second épaulement 19. D'autres moyens de fractionnement de dose sont envisageables.

En référence aux figures 3 à 5, le support de canule 9 comporte un alésage axial central 92 dont le diamètre interne d1 peut être égal ou légèrement supérieur au diamètre externe de ladite canule 4. A son extrémité axiale distale, ledit support de canule 9 comporte une projection radiale 90 s'étendant radialement vers l'intérieur, dont le diamètre interne d2 est égal ou très légèrement inférieur au diamètre externe de ladite canule 4, d2 étant inférieur à d1. Ainsi, l'insertion de la canule 4 dans le support de canule 9 est aisé, car des frottements importants entre ledit support de canule 9 et ladite canule 4 ne se produisent qu'au niveau de ladite projection radiale 90.

Par ailleurs, cette projection radiale 90 coopère, après insertion de la canule 4, de manière étanche avec ladite canule 4, empêchant ainsi tout liquide ayant fui du réservoir 10 de se propager au-delà de ladite projection 90. Le volume de l'espace susceptible de recevoir du liquide de fuite est donc considérablement réduit par rapport au dispositif de l'art antérieur représenté sur la figure 2.

Avantageusement, la surface radiale d'extrémité frontale 95 de l'extrémité axiale distale du support de canule 9 est parfaitement plane et coopère, lors de l'actionnement, de manière étanche avec le bouchon 20. Ceci limite aussi les risques de fuites, et le volume de telles fuites.

La figure 6 illustre une variante de réalisation, dans laquelle le support de canule 9 est réalisé en deux parties, une partie supérieure 97 formant la majeure partie du support de canule, avec un alésage axial central 92 de diamètre interne constant, et une partie inférieure 96, comportant la projection radiale 90 de diamètre interne réduit. Cette mise en oeuvre simplifie la fabrication car elle ne nécessite pas de contre-dépouille. La partie inférieure 97 peut être fixée à la partie supérieure 96 par tout moyen approprié, tel qu'un collage ou un soudage, ou ces deux parties peuvent être co-moulées ou surmoulées l'une sur l'autre.

Le tableau ci-dessous montre les performances améliorées de l'invention, par rapport à un dispositif standard selon la figure 2.

| Poids de dose Remplissage 220 µl | Moyenne Somme des doses (mg) | Ecart-type Somme des doses | Cp | Cpk | Nombre de doses hors specs Somme des dose | Nombre de fuites |
|---|---|---|---|---|---|---|
| Bidose avec canule standard | 187,2 | 8,1 | 0,8 | 0,3 | 2/30 | 8/30 |
| Bidose avec support de canule avec fonction étanchéité | 187,0 | 2,8 | 2,4 | 0,8 | 0/30 | 8/30 |

Dans ce tableau:
Nombre d'échantillons testés: 30
Cp et Cpk sont des critères statistiques:
Cp : différence entre la limite supérieure et la limite inférieure dans l'espace, multipliée par un sixième des déviations ; plus le Cp est élevé, moins les fuites seront dispersives; le Cp ne tient pas compte de l'endroit où la distribution est centrée.
Cpk : indice de capabilité pour tester la qualité d'un processus, en admettant certaines limites de spécifications ; il est toujours utilisé en conjonction avec le Cp ; le Cpk considère l'endroit où la distribution est centrée ; plus le Cpk est élevé, plus la dose sera à l'intérieure de la spécification.
Spec: spécification; la specification de dose est 200 ± 10% (soit entre 180 et 220) sur la moyenne, 200 ± 15% (soit entre 170 et 230) sur les doses individuelles.

Ce tableau démontre que la présente invention améliore les performances du dispositif, en réduisant les fuites, et ainsi en augmentant la précision et la reproductibilité du dosage.

On constate que l'invention donne de meilleures performances avec l'écart-type qui est nettement plus faible, des valeurs Cp et Cpk supérieures, et un nombre de doses hors spécification minimal.

La présente invention a été décrite en référence à des modes de réalisation avantageux, mais il est entendu qu'un homme du métier peut y apporter toutes modifications, sans sortir du cadre de la présente invention tel que défini par les revendications annexées.

## Revendications

1. Dispositif de distribution de produit fluide comportant un corps (1) et une tête de distribution (2) pourvue d'un orifice de distribution (3) et déplaçable axialement par rapport audit corps (1) lors de l'actionnement, ledit corps (1) recevant un réservoir (10) contenant du produit fluide, ledit réservoir (10) comportant un tube creux (11) ayant une ouverture axiale proximale (12) fermée par un bouchon (20) adapté à coulisser de manière étanche dans ledit tube (11) lors de l'actionnement, ladite tête de distribution (2) comportant une canule creuse (4) reliée d'un côté audit orifice de distribution (3) et pourvue de l'autre côté d'une pointe de perçage (5) pourvue d'une ouverture (6) pour percer ledit bouchon (20), ladite canule (4) étant insérée dans un support de canule (9) solidaire de ladite tête de distribution (2), ledit support de canule (9) comportant un alésage axial central (92) dont le diamètre interne (d1) est égal ou légèrement supérieur au diamètre externe de ladite canule (4), **caractérisé en ce que** l'extrémité axiale distale dudit support de canule (9) comporte une projection radiale (90) s'étendant radialement vers l'intérieur, dont le diamètre interne (d2) est très légèrement inférieur au diamètre externe de ladite canule (4), le diamètre interne (d2) de ladite projection radiale (90) étant inférieur au diamètre interne (d1) dudit alésage axial central (92).

2. Dispositif selon la revendication 1, dans lequel, après insertion de la canule (4) dans le support de canule (9), ladite projection radiale (90) coopère de manière étanche avec ladite canule (4).

3. Dispositif selon la revendication 1 ou 2, dans lequel la surface radiale d'extrémité frontale (95) de l'extrémité axiale distale du support de canule (9) est plane et coopère, lors de l'actionnement, de manière étanche avec le bouchon (20).

4. Dispositif selon l'une quelconque des revendications précédentes, dans lequel le support de canule (9) est réalisé en deux parties, une partie supérieure (97) avec un alésage axial central (92) de diamètre interne constant, et une partie inférieure (96) comportant ladite projection radiale (90) de diamètre interne réduit.

5. Dispositif selon l'une quelconque des revendications précédentes, dans lequel ledit réservoir (10) est formé par un tube creux borgne (11).

6. Dispositif selon l'une quelconque des revendications précédentes, dans lequel ledit réservoir (10) contient une dose unique de produit fluide distribuée lors d'un seul actionnement du dispositif.

7. Dispositif selon l'une quelconque des revendications 1 à 5, dans lequel ledit réservoir (10) contient deux doses de produit fluide distribuées lors de deux actionnements successifs du dispositif.

## Patentansprüche

1. Ausgabevorrichtung für ein fluides Produkt, aufweisend einen Körper (1) und einen Ausgabekopf (2), der mit einer Ausgabeöffnung (3) versehen und bei Betätigung axial in Bezug auf den Körper (1) verschiebbar ist, wobei der Körper (1) einen Behälter (10) aufnimmt, der fluides Produkt enthält, wobei der Behälter (10) ein Hohlrohr (11) aufweist, das eine axiale proximale Öffnung (12) umfasst, die durch einen Pfropfen (20) verschlossen ist, der dazu geeignet ist, bei Betätigung dichtend in dem Rohr (11) zu gleiten, wobei der Ausgabekopf (2) eine Hohlnadel (4) aufweist, die einerseits mit der Ausgabeöffnung (3) verbunden und andererseits mit einer Durchstechspitze (5) mit einer Öffnung (6) versehen ist, um den Pfropfen (20) zu durchstechen, wobei die Hohlnadel (4) in einem Hohlnadelhalter (9) eingefügt ist, der mit dem Ausgabekopf (2) einstückig ausgebildet ist, wobei der Hohlnadelhalter (9) eine axiale mittige Ausbohrung (92) aufweist, deren Innendurchmesser (d1) gleich oder etwas größer als der Außendurchmesser der Hohlnadel (4) ist, **dadurch gekennzeichnet, dass** das axiale distale Ende des Hohlnadelhalters (9) einen radialen Vorsprung (90) aufweist, der sich radial nach innen erstreckt und dessen Innendurchmesser (d2) nur ganz leicht kleiner als der Außendurchmesser der Hohlnadel (4) ist, wobei der Innendurchmesser (d2) des radialen Vorsprungs (90) kleiner als der Innendurchmesser (d1) der axialen mittigen Ausbohrung (92) ist.

2. Vorrichtung nach Anspruch 1, wobei der radiale Vorsprung (90) nach dem Einfügen der Hohlnadel (4) in den Hohlnadelhalter (9) dichtend mit der Hohlnadel (4) zusammenwirkt.

3. Vorrichtung nach Anspruch 1 oder 2, wobei die radiale Oberfläche des vorderen Endes (95) des axialen distalen Endes des Hohlnadelhalters (9) eben ist und bei Betätigung dichtend mit dem Pfropfen (20) zusammenwirkt.

4. Vorrichtung nach einem der vorhergehenden Ansprüche, wobei der Hohlnadelhalter (9) zweiteilig gestaltet ist, wobei ein oberer Teil (97) eine axiale mittige Ausbohrung (92) von gleichbleibendem Innendurchmesser aufweist und ein unterer Teil (96) den radialen Vorsprung (90) von verringertem Innendurchmesser aufweist.

5. Vorrichtung nach einem der vorhergehenden Ansprüche, wobei der Behälter (10) durch ein geschlossenes Hohlrohr (11) gebildet ist.

6. Vorrichtung nach einem der vorhergehenden Ansprüche, wobei der Behälter (10) eine einzelne Dosis fluides Produkt enthält, die bei einer einzigen Betätigung der Vorrichtung ausgegeben wird.

7. Vorrichtung nach einem der Ansprüche 1 bis 5, wobei der Behälter (10) zwei Dosen fluides Produkt enthält, die bei zwei aufeinanderfolgenden Betätigungen der Vorrichtung ausgegeben werden.

## Claims

1. A fluid dispenser device comprising a body (1), and a dispenser head (2) that is provided with a dispenser orifice (3) and that is axially movable relative to said body (1) during actuation, said body (1) receiving a reservoir (10) containing fluid, said reservoir (10) comprising a hollow tube (11) having a proximal axial opening (12) that is closed by a stopper (20) that is adapted to slide in leaktight manner in said tube (11) during actuation, said dispenser head (2) including a hollow cannula (4) that is connected at one end to said dispenser orifice (3), and that is provided at its other end with a perforating tip (5) provided with an opening (6) for perforating said stopper (20), said cannula (4) being inserted into a cannula support (9) that is secured to said dispenser head (2), said cannula support (9) including a central axial borehole (92) having an inside diameter (d1) that is equal to, or a little greater than, the outside diameter of said cannula (4), the device being **characterized in that** the distal axial end of said cannula support (9) includes a radial projection (90) that extends radially inwards, having an inside diameter (d2) that is a little less than the outside diameter of said cannula (4), the inside diameter (d2) of said radial projection (90) being less than the inside diameter (d1) of said central axial borehole (92).

2. A device according to claim 1, wherein, after inserting the cannula (4) into the cannula support (9), said radial projection (90) co-operates in leaktight manner with said cannula (4).

3. A device according to claim 1 or claim 2, wherein the front-end radial surface (95) of the distal axial end of the cannula support (9) is plane, and during actuation it co-operates in leaktight manner with the stopper (20).

4. A device according to any preceding claim, wherein the cannula support (9) is made of two portions, a top portion (97) with a central axial borehole (92) of inside diameter that is constant, and a bottom portion (96) including said radial projection (90) of inside diameter that is smaller.

5. A device according to any preceding claim, wherein said reservoir (10) is formed by a blind hollow tube (11).

6. A device according to any preceding claim, wherein said reservoir (10) contains a single dose of fluid for dispensing during a single actuation of the device.

7. A device according to any one of claims 1 to 5, wherein said reservoir (10) contains two doses of fluid for dispensing during two successive actuations of the device.
